# EUROPÄISCHE PATENTANMELDUNG

(11) **EP 1 308 421 A2**
(43) Veröffentlichungstag der Anmeldung: **07.05.2003**
(21) Anmeldenummer: 02024277.2
(22) Anmeldetag: 31.10.2002
(51) Int. Cl.: C02F 1/461

(54) **Vorrichtung zur elektrochemischen Behandlung einer Flüssigkeit und Betriebsverfahren einer solchen Vorrichtung**

(30) Priorität: 02.11.2001 DE 10153897
(71) Anmelder: SCHÖBERL, Meinolf, D-83209 Prien (DE)
(72) Erfinder: SCHÖBERL, Meinolf, D-83209 Prien (DE)
(74) Vertreter: Schlimme, Wolfram, Dr.-Ing.

(57) **Zusammenfassung**

Eine Vorrichtung zur elektrochemischen Behandlung einer Flüssigkeit, insbesondere zur Entkeimung von Wasser ist versehen mit einem rohrförmigen Gehäusemantel, der zumindest auf seiner Innenseite als erster Elektrodenkörper (2) ausgebildet ist, einem zentralen axialen zweiten Elektrodenkörper (4), einem ersten und zweiten Deckelteil (7, 8) welches jeweils ein axiales Ende des rohrförmigen Gehäusemantels verschließt, so daß zwischen den beiden Elektrodenkörpern (2, 4) ein Ringspaltraum (6) gebildet ist, einem am ersten axialen Ende vorgesehenen Einlaßkanal (9) für die Flüssigkeit, der in den Ringspaltraum (6) mündet, einem am zweiten axialen Ende vorgesehenen Auslaßkanal (12) für die Flüssigkeit, der ebenfalls in den Ringspaltraum (6) mündet und einer Stromversorgungseinheit (40), die elektrisch mit dem ersten und zweiten Elektrodenkörper (2, 4) verbunden ist, wobei ein Elektrodenkörper als Anode (A) und der andere Elektrodenkörper als Kathode (K) bestimmt ist. Bei einer derartigen Vorrichtung mündet der Auslaßkanal (12) im Bereich der Oberfläche (4') des als Anode (A) bestimmten Elektrodenkörpers in den Ringspaltraum (6) und weist dazu zumindest eine Mündung auf.

## Beschreibung

Die Erfindung betrifft eine Vorrichtung zur elektrochemischen Behandlung einer Flüssigkeit, insbesondere zur Entkeimung von Wasser gemäß dem Oberbegriff des Patentanspruchs 1. Sie betrifft weiterhin eine verfahrenstechnische Anordnung unter Verwendung einer derartigen Vorrichtung sowie ein Verfahren zum Betrieb einer solchen verfahrenstechnischen Anordnung.

Eine gattungsgemäße Vorrichtung zur elektrochemischen Behandlung einer Flüssigkeit ist aus DE 35 23 026 A1 bekannt. Die dort als Oxidationskammer bezeichnete Vorrichtung weist einen Ringspaltraum zwischen einer zentralen Anode und einer als ringförmigem Mantel ausgebildeten Kathode auf, durch welchen Wasser geleitet wird. Aufgrund der an die Anode und Kathode angelegten elektrischen Spannung wird das durch den Ringspaltraum fließende Wasser nach dem Prinzip der anodischen Oxidation entkeimt und aufbereitet.

Eine Begleiterscheinung dieser elektrochemischen Behandlung des Wassers ist die Ablagerung von Kalk oder anderen im Wasser enthaltenen Mineralstoffen auf der Kathode, was im Extremfall dazu führen kann, daß sich der Ringspaltraum mit abgelagertem Kalk zusetzt und somit eine Durchströmung des Ringspaltraums erschwert oder unmöglich gemacht wird.

Um diese Ablagerungen auf der Kathode zu vermeiden hat man bereits versucht, die Kathodenoberfläche während des Betriebs mechanisch zu reinigen. So ist in der DE 37 08 947 A1 eine ähnliche Oxidationskammer offenbart, bei der die zentrale Anode in der Vorrichtung zur elektrochemischen Behandlung angetrieben drehbar angeordnet und auf ihrem Umfang mit achsparallel verlaufenden Schabern versehen ist, die bei Rotation der Anode Ablagerungen von der Kathodenoberfläche abschaben. Eine derartige Konstruktion ist jedoch technisch äußerst aufwendig und daher teuer in der Herstellung und zudem wartungsaufwendig.

Ziel der vorliegenden Erfindung ist es, eine gattungsgemäße Vorrichtung zur elektrochemischen Behandlung einer Flüssigkeit, insbesondere zur Entkeimung von Wasser, anzugeben, die bei einfachem und somit kostengünstigem Aufbau über einen vorgegebenen Zeitraum wartungsfrei betreibbar ist. Diese Aufgabe wird gemäß Patentanspruch 1 dadurch gelöst, daß der Auslaßkanal im Bereich der Oberfläche des als Anode bestimmten Elektrodenkörpers in den Ringspaltraum mündet.

Durch diese besondere Anordnung der Mündung des Auslaßkanals im Bereich der Oberfläche des als Anode bestimmten Elektrodenkörpers wird auf zuverlässige Weise der engste, einer Verkalkung potentiell ausgesetzte Leitungsquerschnitt in der Vorrichtung vor einem Zusetzen durch Kalkablagerungen bewahrt. Versuche des Patentanmelders haben diese im Anspruch 1 beanspruchte Lage der Mündung des Auslaßkanals als optimale Lage zur Verkaltungsverhinderung ergeben.

Besonders vorteilhaft ist dabei, wenn die zumindest eine Mündung des Auslaßkanals in dem Bereich der Oberfläche des als Anode bestimmten Elektrodenkörpers in den Ringspaltraum mündet, dem, in Radialrichtung gesehen, zumindest teilweise ein Bereich der Oberfläche des als Kathode bestimmten Elektrodenkörpers gegenüber liegt.

Insbesondere vorteilhaft ist es, wenn die zumindest eine Mündung des Auslaßkanals in den Ringspaltraum in der Oberfläche des als Anode ausgebildeten Elektrodenkörpers vorgesehen ist.

Vorzugsweise kann aber auch die zumindest eine Mündung des Auslaßkanals in den Ringspaltraum der Oberfläche des als Anode bestimmten Elektrodenkörpers in unmittelbarer Nähe benachbart gelegen sein.

In einer bevorzugten Ausführungsform ist die erfindungsgemäße Vorrichtung so ausgestaltet, daß der zentrale axiale Elektrodenkörper als Anode bestimmt ist und daß der rohrförmige Elektrodenkörper als Kathode bestimmt ist. Bei dieser Ausgestaltung ist es vorteilhaft, daß die Bestimmung des rohrförmigen Elektrodenkörpers als Kathode, die die zentrale Anode koaxial umgibt, für eine gegenüber der Anodenoberfläche wesentliche größere Kathodenoberfläche sorgt. Damit lagern sich die Kalkabscheidungen auf der im Vergleich zur Oberfläche der zentralen stabförmigen Elektrode (Anode) wesentliche größeren Oberfläche der diese umgebenden rohrförmigen Elektrodenoberfläche (Kathode) ab, so daß die Geschwindigkeit des Aufbaus einer Kalkschicht in Radialrichtung (zur Anode hin) gegenüber der anderen Alternative mit zentraler stabförmiger Kathode wesentlich reduziert ist.

Zusätzlich ergibt sich durch diese Elektrodenanordnung eine im Vergleich zur Stromdichte an der zentralen Anode deutlich niedrigere kathodische Stromdichte, was zu einer sehr vorteilhaften langsamen Reaktionsgeschwindigkeit der Kalkabscheidung führt.

In einer weiteren bevorzugten Ausführungsform ist der radiale Abstand zwischen der Anodenoberfläche und der Kathodenoberfläche so groß dimensioniert, daß in einem vorgegebenen Betriebszeitraum, auch unter ungünstigsten Betriebsbedingungen, keine Kalkbrücken zwischen Anode und Kathode entstehen können. Derartige ungünstigste Betriebsbedingungen sind beispielsweise definiert durch eine sehr hohe Wasserhärte, eine sehr schlechte Leitfähigkeit des Wassers und/oder eine niedrige Chlorid-Konzentration im Wasser.

Weiter vorzugsweise ist der Einlaßkanal derart ausgebildet, daß er in etwa tangential zur Oberfläche der Anode und/oder der Kathode und vorzugsweise in einem Winkel zur Längsachse der Vorrichtung in den Ringspaltraum mündet, um der Flüssigkeitsströmung im Ringspaltraum einen Drall zu vermitteln. Diese Drallbeaufschlagung der in den Ringspaltraum eintretenden Strömung hat sich als besonders vorteilhaft erwiesen, da hierdurch der gezielte Kalkaufbau auf der Kathodenoberfläche in Umfangsrichtung homogener erfolgt als bei nicht drallbehafteter Einströmung.

Dabei hat sich insbesondere als vorteilhaft erwiesen, wenn die Querschnittsfläche der Mündung(en) des Einlaßkanals so dimensioniert ist, daß die Einströmgeschwindigkeit der Flüssigkeit in den Ringspaltraum höher ist als die aufgrund der Querschnittsfläche des Ringspalts und des diesen durchströmenden Flüssigkeitsvolumens pro Zeiteinheit definierte axiale Durchströmgeschwindigkeit. Vorzugsweise liegt die Einströmgeschwindigkeit um ein Vielfaches über dieser axialen Durchströmgeschwindigkeit.

Eine verfahrenstechnische Anordnung zur elektrochemischen Behandlung einer Flüssigkeit, insbesondere zur Entkeimung von Wasser, mit einer erfindungsgemäßen Vorrichtung kennzeichnet sich dadurch, daß der Auslaßkanal der Vorrichtung über eine Fluidleitung mit dem Eingang eines Filters verbunden ist, daß der Ausgang des Filters mit einer Leitung verbunden ist, die in einen Pufferbehälter für die Flüssigkeit mündet und daß der Verbraucher aus dem Pufferbehälter durch eine Entnahmeleitung gespeist wird. Bei dieser Anordnung werden eventuell aus dem Ringspalt ausgetragene Ablagerungspartikel im Filter abgefangen. Die Speisung des Verbrauchers aus dem Pufferbehälter sorgt zudem dafür, daß ein kontinuierlicher Betrieb der Vorrichtung mit konstantem, optimiertem Volumenstrom ermöglicht wird, auch wenn ein spontaner hoher Flüssigkeitsverbrauch auftritt.

Besonders vorteilhaft ist es, wenn eine Rücklaufleitung von der Entnahmeleitung, vorzugsweise kurz vor dem Verbraucher, abzweigt und zum Einlaßkanal der Vorrichtung zur elektrochemischen Behandlung führt und wenn ein Ventil vorgesehen ist, welches den Durchfluß durch die Rücklaufleitung freigibt oder sperrt. Diese Ausbildung der verfahrenstechnischen Anordnung gestattet es, dafür zu sorgen, daß der Anteil an in der Vorrichtung erzeugten, auf die Flüssigkeit einwirkenden Reagenzien, zum Beispiel freie Oxidantien, im Pufferbehälter stets ausreichend hoch ist, um beispielsweise eine Nachverkeimung der im Pufferbehälter und in den Versorgungsleitungen zu einzelnen Verbrauchern vorhandenen Flüssigkeit über einen längeren Zeitraum zu unterbinden.

Alternativ dazu kann in der Entnahmeleitung, vorzugsweise kurz vor dem Verbraucher eine von einem Ventil absperrbare Auslaßvorrichtung vorgesehen sein, um Flüssigkeit aus dem Pufferbehälter und der Entnahmeleitung in einen Abfluß zu leiten. Diese etwas einfachere Ausgestaltung der verfahrenstechnischen Anordnung verzichtet gegenüber der vorherigen Ausgestaltung auf die Rückführung der Flüssigkeit zur Vorrichtung zur elektrochemischen Behandlung, so daß bei dieser Ausgestaltung stets frisch aufbereitetes Wasser durch die Vorrichtung und das Filter in den Pufferbehälter geleitet wird.

Die erfindungsgemäße verfahrentechnische Anordnung wird bevorzugt in einem Verfahren betrieben, bei welchem Flüssigkeit, insbesondere Wasser, aus einem Wasservorrat oder von einem vorhandenen Wasserleitungssystem zunächst durch die Vorrichtung zur elektrochemischen Behandlung geleitet wird, wobei an deren Anode und Kathode eine elektrische Spannung angelegt ist, bei welchem die aus der Vorrichtung austretende Flüssigkeit durch ein Filter geleitet wird, bei welchem die aus dem Filter austretende Flüssigkeit in einen Pufferbehälter geleitet wird und bei welchem ein Verbraucher aus dem Flüssigkeitsvorrat im Pufferbehälter gespeist wird und welches sich dadurch auszeichnet, daß der Flüssigkeitsinhalt des Pufferbehälters und vorzugsweise auch der Entnahmeleitung intervallartig durch aus der Vorrichtung neu zugeführte Flüssigkeit ersetzt wird.

Die Erfindung wird nachfolgend anhand eines Beispiels unter Bezugnahme auf die Zeichnung näher erläutert; in dieser zeigt:
- **Fig. 1.**: eine teilweise geschnittene Ansicht der erfindungsgemäßen Vorrichtung und
- **Fig. 2.**: ein Schaubild einer verfahrenstechnischen Anordnung gemäß der Erfindung.

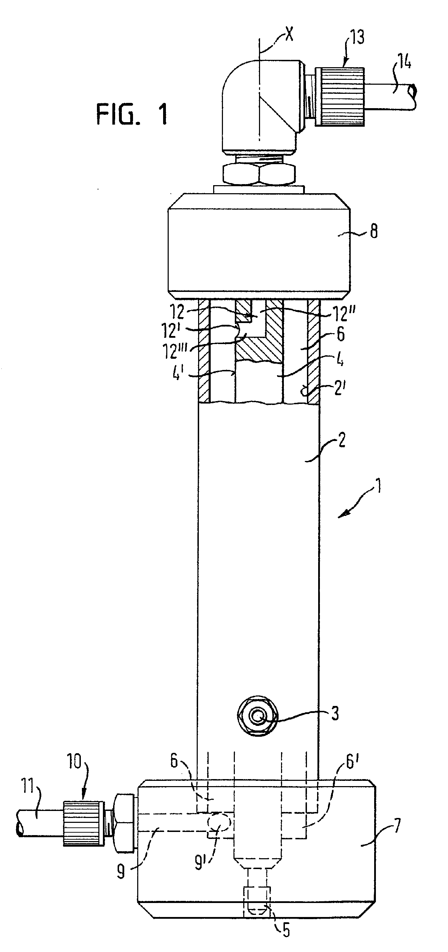

In Fig. 1 ist eine Vorrichtung 1 zur elektrochemischen Behandlung einer Flüssigkeit, insbesondere zur Entkeimung von Wasser gezeigt. Bei dieser Vorrichtung handelt es sich um einen sogenannten Rohrreaktor zur anodischen Oxidation. Ein rohrförmiger äußerer Elektrodenkörper 2 ist mittels eines ersten elektrischen Anschlußteils 3 mit einer Stromversorgungseinheit 40 elektrisch verbunden.

Koaxial innerhalb des rohrförmigen äußeren Elektrodenkörpers 2 ist ein stabartiger runder innerer Elektrodenkörper 4 vorgesehen, der über ein zweites elektrisches Anschlußteil 5 ebenfalls mit der Stromversorgungseinheit 40 elektrisch verbunden ist.

Die beiden Elektrodenkörper 2, 4 sind dabei so an die Stromversorgungseinheit angeschlossen, daß der innere Elektrodenkörper 4 eine Anode A und der äußere Elektrodenkörper eine Kathode K bildet.

Zwischen der radial inneren Oberfläche 2' des rohrförmigen äußeren Elektrodenkörpers 2 und der radial äußeren Oberfläche 4' des stabartigen inneren Elektrodenkörpers 4 ist ein Ringspaltraum 6 gebildet.

Der einen rohrförmigen Gehäusemantel der Vorrichtung 1 bildende äußere Elektrodenkörper 2 ist an seinen axialen Enden jeweils mit einem Deckelteil 7, 8 verschlossen. Die beiden Deckelteile 7, 8 dienen außerdem zur Lagerung des stabartigen inneren Elektrodenkörpers 4.

Das erste, in Fig. 1 untere, Deckelteil 7 ist mit einem Einlaßkanal 9 versehen, der im wesentlichen tangential in eine untere Fortsetzung 6' des Ringspaltraums 6 mündet. Der Einlaßkanal 9 ist über ein Anschlußstück 10 mit einer Einlaßleitung 11 verbunden.

Am in Fig. 1 oberen Ende des rohrförmigen äußeren Elektrodenkörpers 2 ist das zweite, obere Deckelteil 8 vorgesehen, durch welches der stabartige innere Elektrodenkörper 4 in Axialrichtung mit seinem oberen Ende abgedichtet hindurchgeführt ist. Der stabartige innere Elektrodenkörper 4 ist in diesem Bereich mit einer Axialbohrung 12" versehen, die innerhalb des zweiten Deckelteils 8 oder kurz unterhalb von diesem in eine Radialbohrung 12''' übergeht, welche an der radial äußeren Oberfläche 4' des stabartigen inneren Elektrodenkörpers 4 in den Ringspaltraum 6 mündet. Die Axialbohrung 12" und die Radialbohrung 12''' bilden gemeinsam einen Auslaßkanal 12. Dieser Auslaßkanal 12 ist über ein am oberen freien Ende des stabartigen inneren Elektrodenkörpers 4 angebrachtes Anschlußstück 13 mit einer Auslaßleitung 14 verbunden.

Durch die Einlaßleitung 11 zugeführte Flüssigkeit (beispielsweise Wasser) tritt durch den Einlaßkanal 9 und dessen Mündung 9' in den Ringspaltraum 6 mit im wesentlichen tangentialer Strömungsrichtung ein, so daß sich innerhalb des Ringspaltraums 6 eine von unten nach oben gerichtete Drallströmung ergibt. Die sich im Ringspaltraum 6 nach oben bewegende Flüssigkeit wird der elektrochemischen Wirkung zwischen Anode A und Kathode K ausgesetzt. Die Flüssigkeit steigt bis zur Mündung 12' des Auslaßkanals 12 und tritt durch diesen aus dem Ringspaltraum 6 aus. Sie fließt dann durch das obere Anschlußstück 13 in die Auslaßleitung 14.

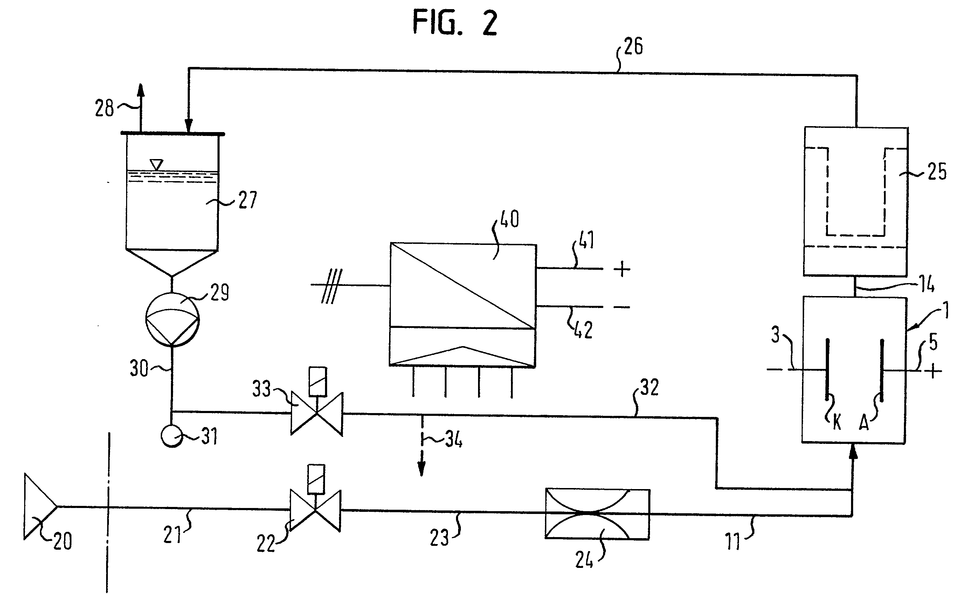

In Fig. 2 ist eine verfahrenstechnische Anordnung dargestellt, die die in Fig. 1 gezeigt Vorrichtung 1 zur elektrochemischen Behandlung einer Flüssigkeit aufweist.

Von einem Flüssigkeits- oder Wasserreservoir oder einem Wasserleitungsanschluß 20 wird das Wasser durch einen ersten Zuleitungsabschnitt 21 einem Absperr-Magnetventil 22 zugeführt. Ist dieses Ventil geöffnet, so fließt das Wasser vom Ventil 22 durch einen zweiten Zuleitungsabschnitt 23 zu einem Durchflußregler 24. Am Ausgang des Durchflußreglers 24 ist die zur Vorrichtung 1 führende Einlaßleitung 11 angeschlossen. Die aus der Vorrichtung 1 herausführende Auslaßleitung 14 leitet das in der Vorrichtung behandelte Wasser zu einem Filter 25, in welchem einerseits Schwebstoffe, wie beispielsweise Kalkpartikel, abgeschieden werden und in welchem andererseits in der Vorrichtung entstandene feine Gasbläschen zu größeren Gasblasen agglomeriert werden, so daß das Filter 25 auch einen wesentlichen Beitrag zur Entgasung des behandelten Wassers leistet. Vom Filter 25 führt eine Verbindungsleitung 26 zu einem Pufferbehälter 27, in welchen das aus dem Filter 25 abgeführte Wasser zusammen mit den dort entstandenen größeren Gasblasen geleitet wird. Die Gasblasen steigen im Pufferbehälter 27 auf und verlassen diesen durch eine Entgasungsleitung 28.

Eine Pumpe 29 ist am Ausgang des Pufferbehälters 27 angeordnet und pumpt bei Bedarf Wasser aus dem Pufferbehälter 27 durch eine Entnahmeleitung 30 zu einem Verbraucher 31, der hier nur schematisch dargestellt ist. Der Verbraucher 31 kann beispielsweise ein Sprühgerät in einer zahnärztlichen Behandlungseinrichtung sein.

Kurz vor dem Verbraucher 31 zweigt eine Rücklaufleitung 32 ab, die über ein weiteres Magnetventil 33 geöffnet werden kann. Die Rücklaufleitung 32 führt zur Einlaßleitung 11 und mündet in diese kurz vor deren Eintritt in die Vorrichtung 1.

Alternativ zur Rücklaufleitung 32 kann hinter dem weiteren Magnetventil 33 auch eine hier gestrichelt gezeichnete Auslaßvorrichtung 34 vorgesehen sein, aus der das Wasser im Pufferbehälter 27 und in der Entnahmeleitung 30 bei Bedarf durch Öffnen des Magnetventils 33 in einen Abfluß abgelassen werden kann.

Eine Stromversorgungseinheit 40 ist über elektrische Anschlußleitungen 41, 42 mit dem elektrischen Anschlußteil 3 der Kathode K beziehungsweise mit dem elektrischen Anschlußteil 5 der Anode A verbunden.

Eine beispielhafte Anwendung für die erfindungsgemäße Vorrichtung ist die Entkeimung und Aufbereitung von Betriebswasser für dentale Behandlungseinheiten. Dabei ist die Vorrichtung in eine Versorgungsleitung für das Betriebswasser eingebaut und wird entweder im Zuflußbetrieb für Frischwasser oder im Umwälzbetrieb innerhalb eines Betriebswasserkreislaufs der dentalen Behandlungseinheit durchströmt. Die Vorrichtung kann auch als zentrale Wasseraufbereitungseinheit für mehrere dentale Behandlungseinheiten vorgesehen sein.

Die Erfindung ist nicht auf die obigen Ausführungsbeispiele beschränkt, die lediglich der allgemeinen Erläuterung des Kerngedankens der Erfindung dienen. Im Rahmen des Schutzumfangs kann die erfindungsgemäße Vorrichtung vielmehr auch andere als die oben beschriebenen Ausgestaltungsformen annehmen. Die Vorrichtung kann hierbei insbesondere Merkmale aufweisen, die eine Kombination aus den jeweiligen Einzelmerkmalen der Ansprüche darstellen.

Bezugszeichen in den Ansprüchen, der Beschreibung und den Zeichnungen dienen lediglich dem besseren Verständnis der Erfindung und sollen den Schutzumfang nicht einschränken.

### Bezugszeichenliste

- 1: Vorrichtung
- 2: rohrförmiger äußerer Elektrodenkörper
- 2': radial innere Oberfläche
- 3: äußeres elektrisches Anschlußteil
- 4: stabartiger innerer Elektrodenkörper
- 4': radial äußere Oberfläche
- 5: elektrisches Anschlußteil
- 6: Ringspaltraum
- 6': untere Fortsetzung des Ringspaltraums
- 7: erstes Deckelteil
- 8: zweites Deckelteil
- 9: Einlaßkanal
- 9': Mündung
- 10: Anschlußstück
- 11: Einlaßleitung
- 12: Auslaßkanal
- 12': Mündung
- 12": Axialbohrung
- 12''': Radialbohrung
- 13: Anschlußstück
- 14: Auslaßleitung
- 20: Flüssigkeitsreservoir
- 21: erster Zuleitungsabschnitt
- 22: Absperr-Magnetventil
- 23: zweiter Zuleitungsabschnitt
- 24: Durchflußregler
- 25: Filter
- 26: Verbindungsleitung
- 27: Pufferbehälter
- 28: Entgasungsleitung
- 29: Pumpe
- 30: Entnahmeleitung
- 31: Verbraucher
- 32: Rücklaufleitung
- 33: Magnetventil
- 34: Auslaßvorrichtung
- 40: Stromversorgungseinheit
- 41: elektrische Anschlußleitung
- 42: elektrische Anschlußleitung

- A: Anode
- K: Kathode
- X: Längsachse

## Patentansprüche

1. Vorrichtung zur elektrochemischen Behandlung einer Flüssigkeit, insbesondere zur Entkeimung von Wasser mit
- einem rohrförmigen Gehäusemantel, der zumindest auf seiner Innenseite als erster Elektrodenkörper (2) ausgebildet ist,
- einem zentralen axialen zweiten Elektrodenkörper (4),
- einem ersten und zweiten Deckelteil (7, 8), welches jeweils ein axiales Ende des rohrförmigen Gehäusemantels verschließt, so daß zwischen den beiden Elektrodenkörpern (2, 4) ein Ringspaltraum (6) gebildet ist,
- einem am ersten axialen Ende vorgesehenen Einlaßkanal (9) für die Flüssigkeit, der in den Ringspaltraum (6) mündet,
- einem am zweiten axialen Ende vorgesehenen Auslaßkanal (12) für die Flüssigkeit, der ebenfalls in den Ringspaltraum (6) mündet und
- einer Stromversorgungseinheit (40), die elektrisch mit dem ersten und zweiten Elektrodenkörper (2, 4) verbunden ist, wobei ein Elektrodenkörper als Anode (A) und der andere Elektrodenkörper als Kathode (K) bestimmt ist,
**dadurch gekennzeichnet ,**
**daß** der Auslaßkanal (12) im Bereich der Oberfläche (4') des als Anode (A) bestimmten Elektrodenkörpers in den Ringspaltraum (6) mündet und dazu zumindest eine Mündung aufweist.

2. Vorrichtung nach Anspruch 1,
**dadurch gekennzeichnet ,**
**daß** die Mündung (12') des Auslaßkanals (12) in dem Bereich der Oberfläche des als Anode (A) bestimmten Elektrodenkörpers in den Ringspaltraum (6) mündet, dem, in Radialrichtung gesehen, zumindest teilweise ein Bereich der Oberfläche des als Kathode (K) bestimmten Elektrodenkörpers gegenüber liegt.

3. Vorrichtung nach Anspruch 1 oder 2,
**dadurch gekennzeichnet,**
**daß** die Mündung (12') des Auslaßkanals (12) in den Ringspaltraum (6) in der Oberfläche des als Anode (A) ausgebildeten Elektrodenkörpers vorgesehen ist.

4. Vorrichtung nach Anspruch 1 oder 2,
**dadurch gekennzeichnet,**
**daß** die Mündung (12') des Auslaßkanals (12) in den Ringspaltraum der Oberfläche des als Anode (A) bestimmten Elektrodenkörpers in unmittelbarer Nähe benachbart gelegen ist.

5. Vorrichtung nach einem der vorhergehenden Ansprüche,
**dadurch gekennzeichnet ,**
- **daß** der zentrale axiale Elektrodenkörper (4) als Anode (A) bestimmt ist und
- **daß** der rohrförmige Elektrodenkörper (2) als Kathode (K) bestimmt ist.

6. Vorrichtung nach einem der vorhergehenden Ansprüche,
**dadurch gekennzeichnet ,**
**daß** der radiale Abstand zwischen der Anodenoberfläche (4') und der Kathodenoberfläche (2') so groß dimensioniert ist, daß in einem vorgegebenen Betriebszeitraum, auch unter ungünstigen Betriebsbedingungen, keine Kalkbrücken zwischen Anode (4) und Kathode (2) entstehen können.

7. Vorrichtung nach einem der vorhergehenden Ansprüche,
**dadurch gekennzeichnet ,**
**daß** der Einlaßkanal (9) derart ausgebildet ist, daß er in etwa tangential zur Oberfläche der Anode (A) und/oder der Kathode (K) und vorzugsweise in einem Winkel zur Längsachse (X) der Vorrichtung in den Ringspaltraum (6) durch zumindest eine Mündung mündet, um der Flüssigkeitsströmung im Ringspaltraum (6) einen Drall zu vermitteln.

8. Vorrichtung nach Anspruch 7,
**dadurch gekennzeichnet ,**
**daß** die Querschnittsfläche der Mündung(en) (9') des Einlaßkanals (9) so dimensioniert ist, daß die Einströmgeschwindigkeit der Flüssigkeit in den Ringspaltraum (6) höher ist als die aufgrund der Querschnittsfläche des Ringspalts (6) und des diesen durchströmenden Flüssigkeitsvolumens pro Zeiteinheit definierte axiale Durchströmgeschwindigkeit.

9. Verfahrenstechnische Anordnung zur elektrochemischen Behandlung einer Flüssigkeit, insbesondere zur Entkeimung von Wasser, mit einer Vorrichtung (1) gemäß einem der vorhergehenden Ansprüche,
- wobei der Auslaßkanal (12) der Vorrichtung (1) über eine Fluidleitung (14) mit dem Eingang eines Filters (25) verbunden ist,
- wobei der Ausgang des Filters (25) mit einer Leitung (26) verbunden ist, die in einen Pufferbehälter (27) für die Flüssigkeit mündet und
- wobei der Verbraucher (31) aus dem Pufferbehälter (26) durch eine Entnahmeleitung (30) gespeist wird.

10. Verfahrenstechnische Anordnung nach Anspruch 9,
**dadurch gekennzeichnet ,**
- **daß** eine Rücklaufleitung (32) von der Entnahmeleitung (30), vorzugsweise kurz vor dem Verbraucher (31), abzweigt und zum Einlaß der Vorrichtung (1) zur elektrochemischen Behandlung führt und
- **daß** ein Ventil (33) vorgesehen ist, welches den Durchfluß durch die Rücklaufleitung (32) freigibt oder sperrt.

11. Verfahrenstechnische Anordnung nach Anspruch 9,
**dadurch gekennzeichnet ,**
**daß** in der Entnahmeleitung (30), vorzugsweise kurz vor dem Verbraucher (31), eine von einem Ventil (33) absperrbare Auslaßvorrichtung (34) vorgesehen ist, um Flüssigkeit aus dem Pufferbehälter (27) und der Entnahmeleitung (30) in einen Abfluß zu leiten.

12. Verfahren zum Betrieb einer verfahrenstechnischen Anordnung nach einem der Ansprüche 9 bis 11,
- wobei Flüssigkeit, insbesondere Wasser, aus einem Wasservorrat oder von einem vorhandenen Wasserleitungssystem zunächst durch die Vorrichtung (1) zur elektrochemischen Behandlung geleitet wird, wobei an deren Anode (A) und Kathode (K) eine elektrische Spannung angelegt ist,
- wobei die aus der Vorrichtung (1) austretende Flüssigkeit durch ein Filter (25) geleitet wird,
- wobei die aus dem Filter (25) austretende Flüssigkeit in einen Pufferbehälter (27) geleitet wird und,
- wobei ein Verbraucher (31) aus dem Flüssigkeitsvorrat im Pufferbehälter (27) gespeist wird,
**dadurch gekennzeichnet ,**
**daß** der Flüssigkeitsinhalt des Pufferbehälters (27) und vorzugsweise auch der Entnahmeleitung (30) intervallartig durch aus der Vorrichtung (1) neu zugeführte Flüssigkeit ersetzt wird.
